# EUROPÄISCHE PATENTSCHRIFT

(11) **EP 1 259 503 B1**
(45) Veröffentlichungstag und Bekanntmachung des Hinweises auf die Patenterteilung: **12.09.2007**
(21) Anmeldenummer: 01919369.7
(22) Anmeldetag: 02.03.2001
(51) Int. Cl.: C07D 339/08, C07C 43/225, C07C 43/12, C07C 41/01, C07B 41/04, C09K 19/30

(54) **HERSTELLUNG VON FLÜSSIGKRISTALLEN MIT CF20-BRÜCKE**
PRODUCING LIQUID CRYSTALS WITH CF2O BOND
PRODUCTION DE CRISTAUX LIQUIDES AVEC PONTS CF20

(30) Priorität: 03.03.2000 DE 10010537; 31.05.2000 DE 10027102
(43) Veröffentlichungstag der Anmeldung: 27.11.2002
(73) Patentinhaber: Merck Patent GmbH, 64293 Darmstadt (DE)
(72) Erfinder: KIRSCH, Peer, 64342 Seeheim-Jugenheim (DE); TAUGERBECK, Andreas, 64285 Darmstadt (DE); PAULUTH, Detlef, 64372 Ober-Ramstadt (DE); BREMER, Matthias, 64295 Darmstadt (DE)
(74) Vertreter: Isenbruck, Günter
(86) Internationale Anmeldenummer: PCT/EP2001/002402
(87) Internationale Veröffentlichungsnummer: WO 2001/064667

(56) Entgegenhaltungen:
- EP-A- 0 844 229
- OKUYAMA, T. ET AL.: "Mechanism of Hydrolysis of 1,3-Thiane Derivatives. Breakdown of the Tetrahedral Intermediate" J. ORG. CHEM., Bd. 51, 1986, Seiten 4988-90, XP002173541
- OKUYAMA ET AL.: "Mechanism of Hydrolysis of 2-tert-Butyl-2-methoxy-1,3-dithiolane." J. AM. CHEM. SOC., Bd. 107, Nr. 14, 1985, Seiten 4224-9, XP002173542
- BELLESIA ET AL.: "gamma-alkylketene dithioacetals from 2,3-dichloroaldehyde dithioacetals" GAZZETTA CHIMICA ITALIANA, Bd. 125, Nr. 10, 1995, Seiten 501-4, XP001010520
- SONDEJ S C ET AL: "Gem-Difluoro compounds: a convenient preparation from ketones and aldehydes by halogen fluoride treatment of 1,3-dithiolanes" JOURNAL OF ORGANIC CHEMISTRY,US,AMERICAN CHEMICAL SOCIETY. EASTON, Bd. 51, Nr. 18, 1986, Seiten 3508-3513, XP002125273 ISSN: 0022-3263

## Beschreibung

Die Erfindung betrifft ein Verfahren zur Herstellung von Verbindungen mit mindestens einer -CF₂-O-Brücke im Molekül.

Flüssigkristalle haben ein breites Anwendungsfeld gefunden, seitdem vor etwa 30 Jahren die ersten kommerziell anwendbaren flüssigkristallinen Verbindungen aufgefunden wurden. Bekannte Anwendungsgebiete sind insbesondere Anzeigedisplays für Uhren oder Taschenrechner, oder große Anzeigetafeln, wie sie in Bahnhöfen, Flughäfen oder Sportarenen verwendet werden. Weitere Anwendungsgebiete sind Displays von tragbaren Computern oder Navigationssystemen sowie Videoapplikationen. Insbesondere für die zuletzt genannten Anwendungen werden hohe Anforderungen an Schaltzeiten und den Kontrast der Abbildungen gestellt.

Um für kommerzielle Anwendungen brauchbar zu sein, müssen die flüssigkristallinen Moleküle bestimmte Eigenschaften aufweisen. Um Geräte mit einem Flüssigkristalldisplay unter unterschiedlichen Klimabedingungen einsetzen zu können, müssen die Moleküle über einen möglichst großen Temperaturbereich, der im Bereich der Raumtemperatur liegt, eine stabile nematische Phase ausbilden. Die Verbindungen müssen also einen niedrigen Schmelzpunkt und einen hohen Klärpunkt besitzen.

Um niedrige Schaltzeiten verwirklichen zu können, müssen die Moleküle eine niedrige Rotationsviskosität aufweisen. So werden für Videoapplikationen Schaltzeiten von weniger als 16.7 ms gefordert. Ferner sollen die flüssigkristallinen Moleküle eine hohe dielektrische Anisotropie aufweisen, so daß nur niedrige Schwellenspannungen erforderlich sind. Dies bedeutet einen niedrigen Energiebedarf, so daß beispielsweise in Laptops kleinere und damit leichtere Akkus verwendet werden können. Für die Gestaltung des Displays sind ferner die Doppelbrechungseigenschaften der Moleküle von Bedeutung, welche Kontrast und nutzbaren Blickwinkel beeinflussen.

Um alle diese Anforderungen gleichzeitig erfüllen zu können, werden keine reinen Substanzen verwendet sondern Gemische, die meist 5 bis 15 unterschiedliche Komponenten umfassen. Dies bedeutet, daß die einzelnen Komponenten miteinander verträglich sein müssen, also sich beispielsweise ausreichend ineinander lösen.

Für moderne Aktivmatrixdisplays wird ein hoher Kontrast der Abbildungen gewünscht. Die flüssigkristallinen Verbindungen müssen daher einen hohen spezifischen Widerstand und eine hohe voltage holding ratio aufweisen.

Als flüssigkristalline Verbindungen mit einem besonders hohen spezifischen Widerstand haben sich Verbindungen erwiesen, die fluorhaltige Gruppen in ihrem Molekülgerüst enthalten. So werden z.B. in der EP 0 844 229 A1 flüssigkristalline Verbindungen beschrieben, die eine -O-CF₂-Brücke enthalten. Zur Herstellung dieser -O-CF₂-Brücke werden verschiedene Verfahren vorgeschlagen. Nach einem der beschriebenen Verfahren wird zunächst ein aromatisches Halogenid in eine Grignard-Verbindung oder in eine lithiierte Verbindung überführt und dann mit Schwefelkohlenstoff in die Dithiocarbonsäure überführt. Die Dithiocarbonsäure wird mit einem Phenol in Gegenwart eines Alkalimetallhydrids und Jod in einen Thioester überführt. Mit einem Fluorierungsmittel wird aus dem Thioester dann die gewünschte -O-CF₂-Brücke gebildet.

Nach einem anderen Verfahren wird vorgeschlagen, zunächst ein Cyclohexanon mit Tris(dimethylamino)phosphan und Dibromdifluormethan umzusetzen, um ein Difluormethylenhexyliden zu erhalten. An dieses wird zunächst Brom addiert und dann durch Reaktion mit einem Phenolat unter gleichzeitiger Abspaltung von Bromwasserstoff unter Ausbildung einer -CF₂-O-Brücke verethert.

Nachteil dieser Verfahren ist, daß die Reaktionsgeschwindigkeiten niedrig, die Ausbeuten unzufriedenstellend und die Aufarbeitung und Reinigung des Produkts aufwendig sind.

Aufgabe der Erfindung ist es daher, ein Verfahren zur Herstellung von Verbindungen mit mindestens einer -CF₂-O-Brücke im Molekül zur Verfügung zu stellen, das mit zufriedenstellender Reaktionsgeschwindigkeit zu guten Ausbeuten führt. Zwischen- und Endprodukte sollen auf einfache Weise zu reinigen sein.

Zur Lösung dieser Aufgabe wird beim erfindungsgemäßen Verfahren zunächst ein Bis(alkylthio)carbenium-Salz in Gegenwart einer Base mit einer mindestens eine Hydroxylgruppe aufweisenden Verbindung umgesetzt und anschließend, vorzugsweise in situ, mit einem Fluorierungsmittel und einem Oxidationsmittel zur Verbindung mit mindestens einer -CF₂-O-Brücke im Molekül oxidativ fluoriert.

Die Bis(alkylthio)carbenium-Salze lassen sich sehr einfach aus den entsprechenden Carbonsäuren oder aktivierten Carbonsäurederivaten herstellen. Geeignete Carbonsäurederivate sind beispielsweise Carbonsäurehalogenide, Carbonsäurepseudohalogenide, Carbonsäuresulfonylate, welche geeignet substituiert sind, beispielsweise ein Trifluormethansulfonylat. Weiter können verwendet werden Carbonsäureanhydride und Alkyl- bzw. Phenylcarbonsäureester. Die Salze fallen in sauberer Form aus der Reaktionslösung aus und können ohne weitere Reinigung in der nächsten Stufe eingesetzt werden.

Das Bis(alkylthio)carbenium-Salz wird durch Reaktion mit der mindestens eine Hydroxylgruppe enthaltenden Verbindung zunächst zum Dithioorthoester umgesetzt. Dieser Dithioorthoester wird im allgemeinen nicht isoliert, sondern sofort weiter umgesetzt. Dabei erfolgt die oxidative Fluorierung zur eine -CF₂-O-Gruppe enthaltenden Verbindung unter sehr milden, leicht basischen Bedingungen und ist daher im Gegensatz zu den konventionellen Methoden mit einer Vielzahl von ungeschützten funktionellen Gruppen, z.B. einer Nitrilgruppe, kompatibel. Als weiterer Vorteil ergibt sich, daß die Stereochemie der Reste, beispielsweise ein cis- oder trans-Cyclohexylenrest, bei der Reaktion erhalten bleibt. Die prinzipiellen Schritte sind in der folgenden Abbildung 1 zusammengefaßt.

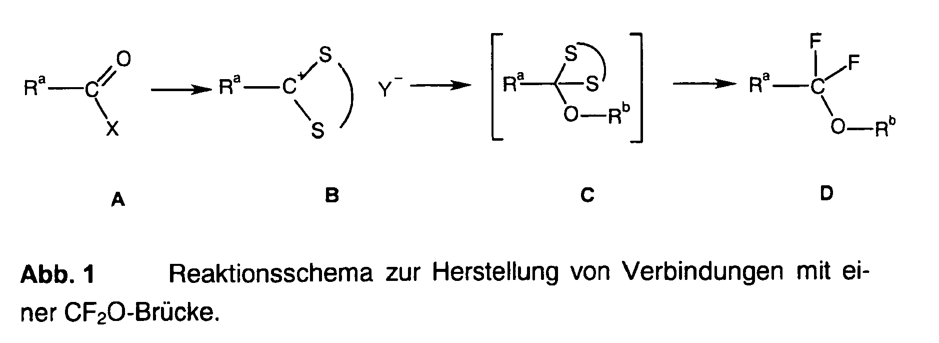

Das Carbonsäurederivat A, in dem X beispielsweise steht für -OH, Halogen, Pseudohalogen, substituiertes Sulfonat, ein Anhydrid, Alkoxy oder Phenoxy, wird mit einem Alkylthiol zum Bis(alkylthio)carbenium-Salz B umgesetzt. Bevorzugt werden Dithiole verwendet, die zur Ausbildung eines zyklischen Kations führen. Insbesondere geeignet sind daher Ethandithiol, Propandithiol oder 1,2-Benzoldithiol, die zur Ausbildung von Dithianylium- bzw. Dithiolanyliumsalzen führen. Dieses Salz B wird anschließend mit einer Hydroxyverbindung R^{b}-OH zum Orthoester C umgesetzt. Der Orthoester C wird im allgemeinen nicht isoliert, sondern direkt oxidativ zur Verbindung D umgesetzt. Das Verfahren ist universell einsetzbar, so daß R^{a} und R^{b} an sich keinen Beschränkungen unterliegen. R^{a} und R^{b} können also unabhängig voneinander beispielsweise ein Alkyl-, ein Aryl-, ein Cycloalkyl oder ein Alkenylrest sein, wobei diese Reste wiederum beliebig substituiert sein können, beispielsweise durch Halogen, Pseudohalogen, Hydroxy- oder Carbonylgruppen.

Für die Herstellung von Flüssigkristallen weist das Bis(alkylthio)carbenium-Salz vorzugsweise eine Struktur der Formel I auf. 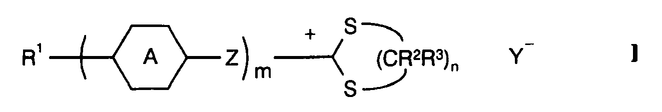 wobei bedeutet 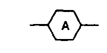 jeweils unabhängig voneinander cis- oder trans-1,4-Cyclohexylen, 1,4-Phenylen oder 1,4-Cyclohex-3-enylen, wobei diese Gruppen auch ein- oder zweifach substituiert sein können durch Halogen, insbesondere Fluor, Pseudohalogen, -OCF₃, -OCHF₂
- R¹: einen unsubstituierten, einen einfach durch -CN oder -CF₃ oder ein mindestens einfach durch Halogen substituierten linearen oder verzweigten Alkyl- oder Alkoxyrest mit 1 bis 12 C-Atomen, einen linearen oder verzweigten Oxyalkyl-, Alkenyl- oder Alkenyloxyrest mit 2 bis 12 C-Atomen oder einen linearen oder verzweigten Oxalkenylrest mit 3 bis 12 C-Atomen, wobei in diesen Resten auch eine oder mehrere CH₂-Gruppen jeweils unabhängig voneinander durch -O-, -S-, -CO-, -CO-O-, -O-CO- oder -O-CO-O- so ersetzt sein können, daß Heteroatome nicht direkt miteinander verknüpft sind, Halogen, vorzugsweise F oder Cl,
- Z: jeweils unabhängig voneinander eine Einfachbindung, eine - CH₂-CH₂-, -CF₂-CF₂-, -CF₂-CH₂-, -CH₂-CF₂-, -CF=CF-, -CH=CH-, -C≡C-, -CO-O-, -O-CO-, -CF₂-O-, -O-CF₂-Gruppe,
- -S-(CR²R³)ₙ-S-: eine Brücke aus zwei Schwefelatomen, die durch eine Kohlenstoffkette mit zwei oder drei Kohlenstoffatomen verbunden ist, wobei die Kohlenwasserstoffbrücke auch einen oder mehrere Substituenten R², R³ tragen kann, insbesondere Alkyl- oder Alkenylgruppen, wobei die Reste R², R³ auch gemeinsam eine Cycloalkylgruppe oder Arylgruppe ausbilden können,
- m: einen ganzzahligen Wert zwischen 0 und 6
- n: 2 oder 3
- Y: ein beliebiges Anion.

Das erfindungsgemäße Verfahren eignet sich allgemein zur Herstellung von Verbindungen mit einer -CF₂-O-Brücke wie z.B. Flüssigkristalle, Vorstufen für Polymere, Pharmazeutika und Pflanzenschutzmittel. Besonders geeignet ist es jedoch zur Herstellung flüssigkristalliner Verbindungen. Da durch das Bis(alkylthio)carbenium-Salz B ein zur einen Seite an die -CF₂-O-Brücke angrenzendes Teilstück in das Molekül eingeführt wird, sind die Reste R¹ bevorzugt als in flüssigkristallinen Verbindungen übliche Strukturelemente ausgebildet. Im folgenden werden in Abb. 2 einige derartiger Teilstücke beispielhaft aufgeführt, wobei diese Aufzählung nicht abschließend ist. R hat dabei die gleiche Bedeutung wie oben für R¹ angegeben.

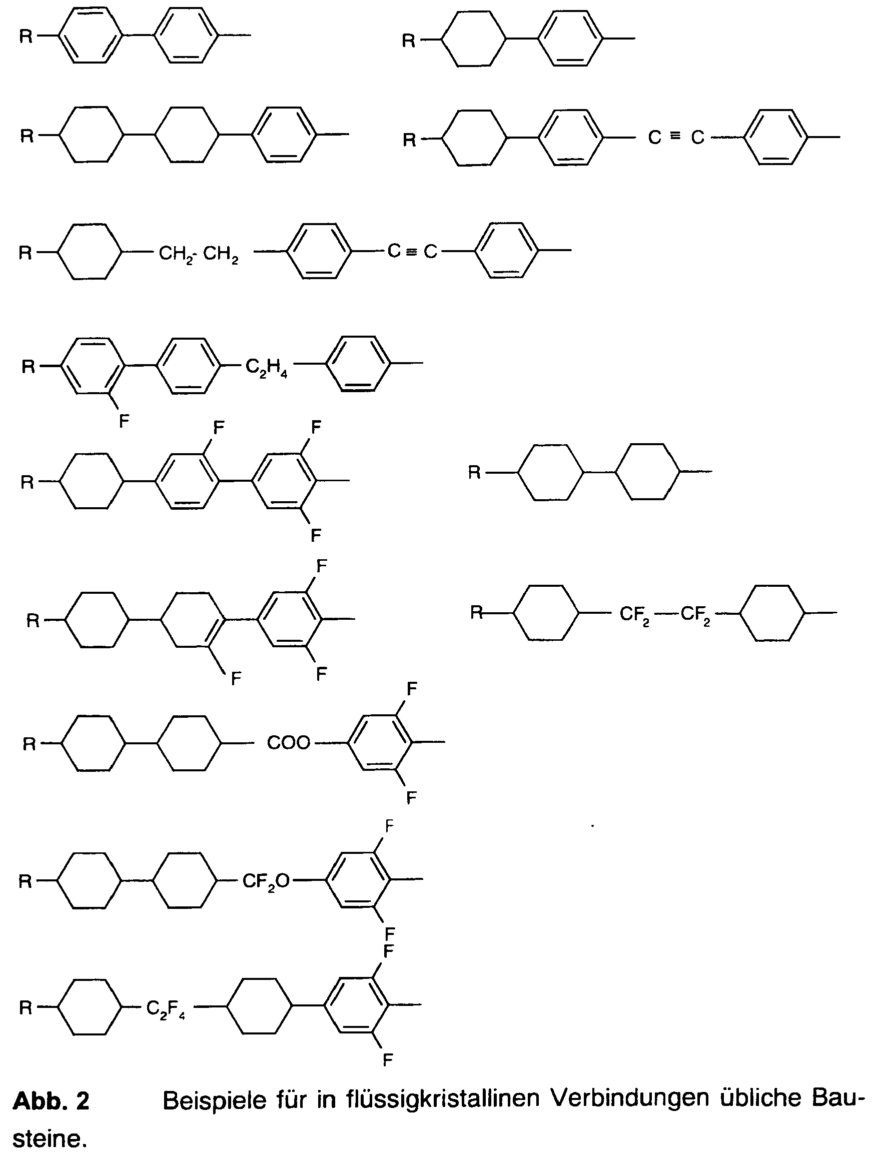

Die mindestens eine Hydroxylgruppe enthaltende Verbindung ist vorzugsweise ein Phenol, das gegebenenfalls substituiert sein kann, insbesondere in 4-Stellung eine polare Gruppe, bevorzugt -F, -Cl, -CN, -NCS, -OCF₃, - OCH₂F oder einen Alkyl-, Cycloalkyl- oder Phenylrest trägt, wobei diese Reste gegebenenfalls wiederum durch Alkyl-, Cycloalkyl- oder Phenylreste substituiert sein können, wobei weiter Wasserstoffatome auch durch Fluor- oder Chloratome substituiert sein können und zwischen den genannten Gruppen jeweils eine -CH₂-CH₂-, -CF₂-CF₂-, -CF₂-CH₂-, -CH₂-CF₂-, -CF=CF-, -CH=CH-, -C≡C-, -COO-, -O-CO-, -O-CF₂- oder -CF₂O-Gruppe vorgesehen sein kann.

Die mindestens eine Hydroxylgruppe enthaltende Verbindung ist in ihrer Struktur an sich keinen besonderen Beschränkungen unterworfen. Da das erfindungsgemäße Verfahren jedoch besonders zur Herstellung von Flüssigkristallen geeignet ist, wird der in der mindestens eine Hydroxylgruppe enthaltenden Verbindung vorgesehene Rest vorzugsweise in der Weise ausgestaltet, daß er in Flüssigkristallen übliche Strukturelemente enthält. Als beispielhafte Strukturelemente können auch hier die in Abbildung 2 gezeigten Bausteine angeführt werden.

Das Bis(alkylthio)carbeniumsalz weist vorzugsweise ein nicht- oder schwach-koordinierendes Anion auf, das insbesondere ausgewählt ist aus der Gruppe, die gebildet ist von Tetrafluoroborat, Hexafluorophosphat, Perchlorat sowie Perfluoralkylsulfonat, insbesondere Trifluormethansulfonat. Diese Salze sind einfach zu verarbeiten, da sie kaum hygroskopisch sind.

Als Oxidationsmittel können übliche Oxidationsmittel verwendet werden. Bevorzugt wird als Oxidationsmittel eine Verbindung eingesetzt, die Haloniumäquivalente freisetzt. Beispielhafte Oxidationsmittel sind N-Bromsuccinimid, N-Jodsuccinimid, 1,3-Dibrom-5,5-dimethylhydanthoin und Brom. Besonders bevorzugt ist Brom, da sich die entstehenden Bromide leicht abtrennen lassen. Ebenfalls geeignet sind beispielsweise SO₂Cl₂, SO₂CIF, Nitrosonium- und Nitroniumsalze sowie Chloramin T.

Als Fluorierungsmittel können übliche Fluorierungsmittel eingesetzt werden. Besonders bevorzugt wird das Fluorierungsmittel ausgewählt aus der Gruppe die gebildet ist von aliphatischen und aromatischen Amin-Fluorwasserstoff-Komplexen, beispielsweise Pyridin-Fluorwasserstoffkomplexe, NEt₃·3HF, 50 % HF in Pyridin, Melamin-HF, Polyvinylpyridin-HF.

Wie bereits erwähnt, eignet sich das erfindungsgemäße Verfahren insbesondere für die Herstellung von Flüssigkristallen. Das erfindungsgemäße Verfahren geht aus von Bis-(alkylthio)carbeniumsalzen. Gegenstand der Erfindung sind daher auch Bis-(alkylthio)carbeniumsalze der Formel I. 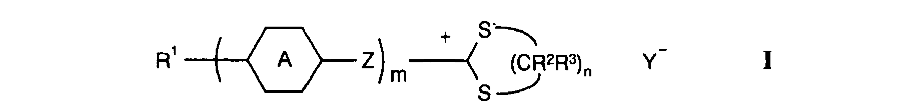 wobei R¹, 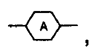 Z, -S-(CR²R³)ₙ-S-, m, n und Y die oben angegebene Bedeutung aufweisen.

Weiter betrifft die Erfindung Dithioorthoester der Formel II 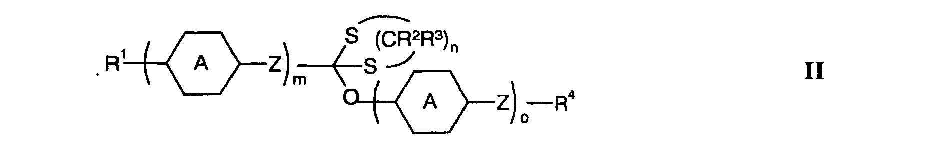 wobei R¹, R², R³, Z, 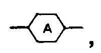 m, n die gleiche Bedeutung haben wie in Anspruch 2 angegeben und R⁴ die gleiche Bedeutung hat wie für R¹ in Anspruch 2 angegeben sowie zusätzlich -H, -F, -Cl, -CN, -SCN, -OCF₃ oder -OCHF₂ bedeuten kann und o ein ganzzahliger Wert zwischen 0 und 6 ist.

Die Erfindung wird anhand von Beispielen näher erläutert.

### Beispiel 1

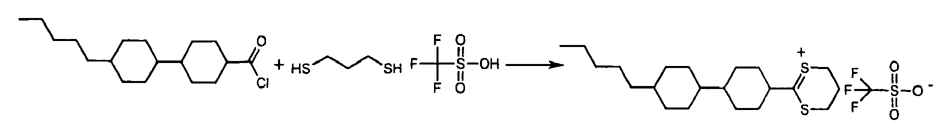

5,9 g 4-(4'-Pentyl-cyclohexyl)cyclohexancarbonsäurechlorid wurden unter Eiskühlung mit 2 ml 1,3-Propandithiol versetzt. Dann wurden 2,6 ml Trifluormethansulfonsäure zugegeben, die Mischung auf 110°C erwärmt und für 15 Minuten gerührt. Der Ansatz wurde aus dem Wärmebad entnommen und etwas abgekühlt. Anschließend wurde in 10 ml Acetonitril gelöst und die Mischung auf 200 ml Ether gegeben. Die ausgefallenen farblosen Kristalle wurden unter Stickstoff abgesaugt und im Vakuum getrocknet.
Ausbeute 3,0 g. Aus der Mutterlauge wurden nach Aufbewahrung bei 0°C weitere 3,1 g Produkt isoliert.
¹³C-NMR (CDCl₃, 303 K): δ_{carbenium} = 203,5 ppm.

### Beispiel 2

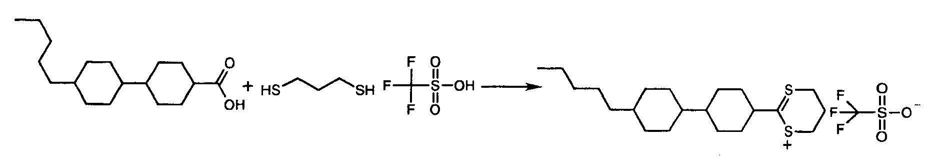

10 g 4-(4'-Pentyl-cyclohexyl)cyclohexancarbonsäure wurden mit 3,6 ml 1,3-Propandithiol versetzt und im Eisbad gekühlt. Es wurden 7,9 ml Trifluormethansulfonsäure zugegeben. Nach erfolgter Zugabe wurde auf 120°C erwärmt und der Ansatz für 30 Minuten gerührt. Der Ansatz wurde aus dem Wärmebad entnommen und in 10 ml Acetonitril gelöst. Die Lösung wurde auf 50 ml eiskalten Ether gegeben, wobei farblose Plättchen ausfielen. Es wurde für weitere 2 Stunden auf - 20°C gekühlt, und dann unter Stickstoff abgesaugt. Nach Trocknung im Vakuum wurden farblose Plättchen erhalten.
Ausbeute: 10,9 g (60,2 % d. Th.)
¹³C-NMR (CDCl₃, 303K): δ_{carbenium} = 203.5 ppm

### Beispiel 3

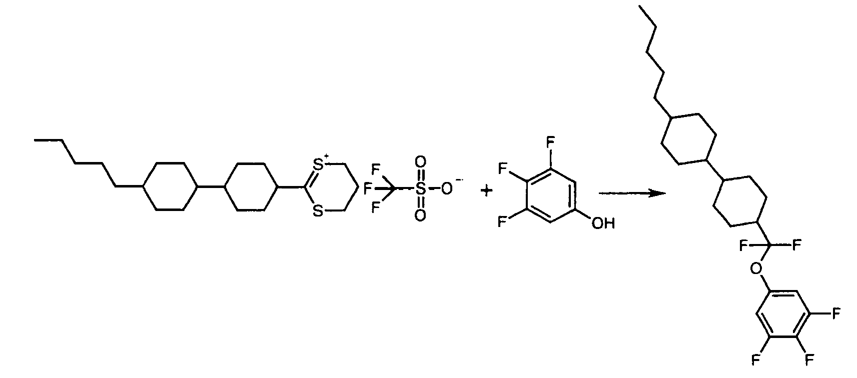

1,06 g des in Beispiel 2 erhaltenen Dithianyliumtrifluormethansulfonats wurden bei - 70°C in 20 ml Dichlormethan vorgelegt und mit einer Mischung aus 0,6 ml Triethylamin und 0,7 g Trifluorphenol (90 % in Toluol) in 2 ml Dichlormethan versetzt, wobei sofort ein farbloser Feststoff ausfiel. Der Ansatz wurde 2 Stunden bei - 70°C gerührt. Anschließend wurden während 20 Minuten 3 g 1,3-Dibrom-5,5-dimethylhydanthoin, suspendiert in 15 ml Dichlormethan, portionsweise zugegeben. Der Ansatz wurde für weitere 90 Minuten bei - 70°C gerührt und dann auf Raumtemperatur erwärmt. Die Lösung wurde unter Rühren auf gesättigte Natriumhydrogencarbonatlösung gegeben, die organische Phase abgetrennt und die wäßrige Phase zweimal mit Dichlormethan extrahiert. Die vereinigten organischen Phasen wurden getrocknet und das Lösungsmittel im Vakuum entfernt. Nach Filtration über Kieselgel wurde ein farbloses Öl erhalten, das langsam durchkristallisierte.
Ausbeute: 415 mg (45,5 % d. Th.)

| | |
|---|---|
| Schmelzpunkt: | 59°C |
| Klärpunkt: | 112°C. |

### Beispiel 4

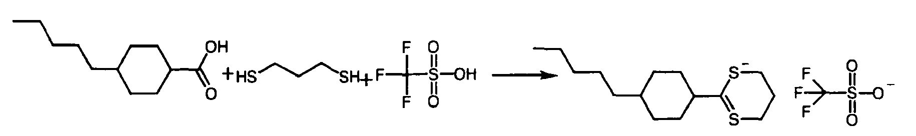

199 g 4-Pentylcyclohexancarbonsäure wurden mit 100 ml 1,3-Propandithiol vermischt und im Eisbad gekühlt. Anschließend wurden 263 ml Trifluormethansulfonsäure zugetropft und nach erfolgter Zugabe der Ansatz für 1 Stunde auf 120°C erwärmt. Nach Abkühlen auf ca. 70°C wurde der Ansatz auf 500 ml eiskalten Dibutylether gegeben und die Lösung über Nacht auf -20°C gekühlt. Die ausgefallenen Kristalle wurden abgesaugt, mit Ether gewaschen und im Vakuum getrocknet.
Ausbeute 92 g (21,9 % d. Th.).

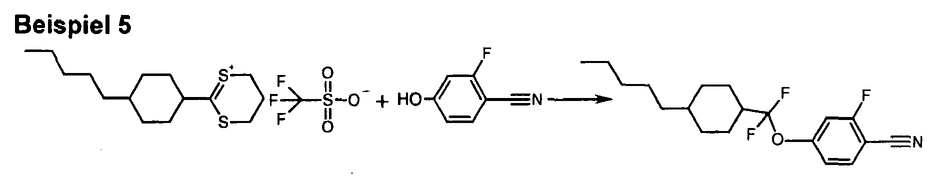

30 g des in Beispiel 4 erhaltenen Dithianyliumtrifluormethansulfonats wurden bei - 70°C in 250 ml Dichlormethan vorgelegt. Es wurde eine Lösung aus 14,8 ml Triethylamin und 12,2 g 3-Fluor-4-cyanophenol in 50 ml Dichlormethan bei - 70°C zugetropft, wobei sofort ein farbloser Feststoff ausfiel. Der Ansatz wurde noch für eine Stunde bei - 70°C gerührt. Es wurden 57,2 ml Triethylamin-tris-hydrofluorid zugetropft und nachdem für weitere 5 Minuten gerührt worden war, wurden 18,2 ml Brom während 45 Minuten bei -70 °C zugetropft. Der Ansatz wurde noch für weitere 60 Minuten gerührt und dann auf Raumtemperatur erwärmt. Die Lösung wurde auf gesättigte Natriumhydrogencarbonatlösung gegeben, die organische Phase abgetrennt und die wäßrige Phase mit Dichlormethan extrahiert. Nach Trocknen der vereinigten organischen Phasen wurde das Lösungsmittel im Vakuum entfernt. Nach Filtration über Kieselgel wurde ein farbloses Öl erhalten.
Ausbeute: 12 g (50 % d. Th.)

### Beispiel 6

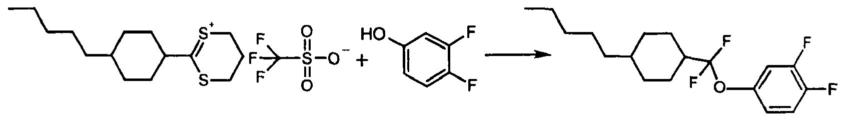

30 g des in Beispiel 4 erhaltenen Dithianyliumtrifluormethansulfonats wurden bei - 70°C in 250 ml Dichlormethan vorgelegt. Es wurde eine Lösung aus 14,8 ml Triethylamin und 11,6 g 3,4-Difluorphenol in 50 ml Dichlormethan bei - 70°C zugetropft, wobei sofort ein farbloser Feststoff ausfiel. Der Ansatz wurde noch für eine Stunde bei - 70°C gerührt. Dann wurden 57,2 ml Triethylamin-tris-hydrofluorid zugetropft und nachdem der Ansatz für weitere 5 Minuten gerührt worden war, 18,2 ml Brom während 45 Minuten zugetropft. Der Ansatz wurde noch 60 Minuten bei - 70°C gerührt und dann auf Raumtemperatur erwärmt. Die Lösung wurde auf Natriumhydrogencarbonatlösung gegeben, die organische Phase abgetrennt und die wäßrige Phase mit Dichlormethan extrahiert. Die vereinigten organischen Phasen wurden getrocknet und das Lösungsmittel entfernt. Nach Filtration über Kieselgel wurde ein farbloses Öl erhalten.
Ausbeute: 8,1 g (34 % d. Th.)

### Beispiel 7

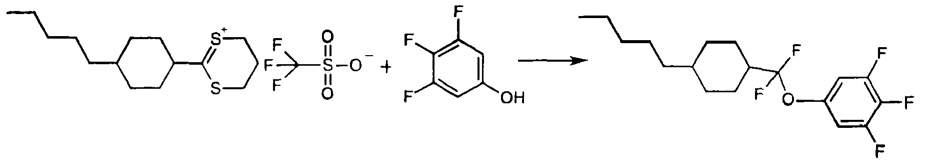

30,0 g des in Beispiel 4 erhaltenen Dithianyliumtrifluormethansulfonats wurden in 250 ml Dichlormethan bei - 70°C vorgelegt. Es wurde eine Lösung aus 14,8 ml Triethylamin und 13,1 g 3,4,5-Trifluorphenol in 50 ml Dichlormethan zugetropft wobei sofort ein farbloser Feststoff ausfiel. Nachdem für eine weitere Stunde bei - 70°C gerührt worden war, wurden 57,2 ml Triethylamin-tris-hydrofluorid zugetropft und nach weiteren 5 Minuten 18,2 ml Brom während 45 Minuten zugetropft. Der Ansatz wurde noch für 60 Minuten gerührt und dann auf Raumtemperatur erwärmt. Die Mischung wurde auf Natriumhydrogencarbonatlösung gegeben, die organische Phase abgetrennt und die wäßrige Phase mit Dichlormethan extrahiert. Die vereinigten organischen Phasen wurden getrocknet und das Lösungsmittel im Vakuum entfernt. Nach Filtration über Kieselgel wurde ein farbloses Öl erhalten.
Ausbeute 7,4 g (30 % d. Th.).

### Beispiel 8

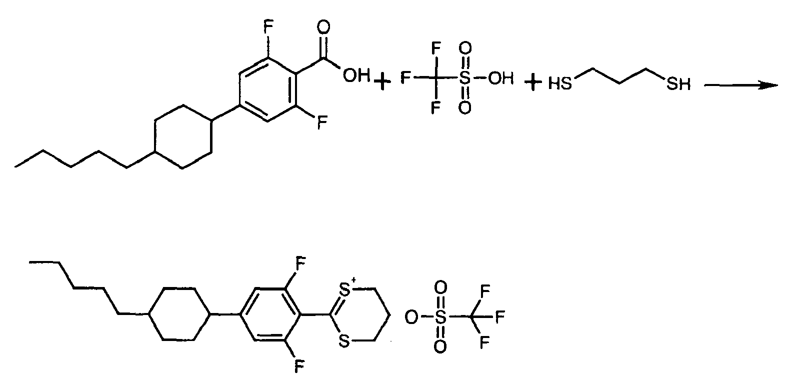

10 g 2,6-Difluor-4-(4-pentylcyclohexyl)benzoesäure, 3,5 ml 1,3-Propandithiol und 8,5 ml Trifluormethansulfonsäure wurden vermischt und 15 min. bei Raumtemperatur sowie 20 min. bei 110°C gerührt. Nach Abkühlen auf Raumtemperatur wurden 80 ml Diethylether zugegeben und der Ansatz bei -20°C über Nacht aufbewahrt. Der ausgefallene Feststoff wurde unter Stickstoff abgesaugt, mit Diethylether gewaschen und im Vakuum getrocknet.
Ausbeute: 10,3 g (60 % d. Th.)
¹³C-NMR (CDCl₃, 303 K): δ_{carbenium} = 212 ppm.

### Beispiel 9

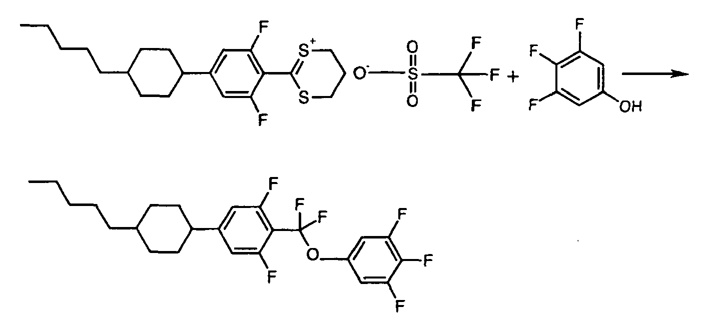

10 g des in Beispiel 8 erhaltenen Feststoffs wurden in 100 ml Dichlormethan gelöst und bei -75°C mit einer Lösung von 6,2 g 3,4,5-Trifluorphenol (90 % in Toluol) und 5,7 ml Triethylamin in 20 ml Dichlormethan versetzt. Die farblose klare Lösung wurde noch 45 min. gerührt und dann tropfenweise mit 15,1 ml Triethylamintrishydrofluorid versetzt. Nach 5 min. wurde eine Suspension von 26,8 g 1,3-Dibrom-5,5-dimethylhydantoin in 50 ml Dichlormethan innerhalb von 45 min. portionsweise zugegeben. Anschließend wurde auf -20°C erwärmt und die orangefarbige Suspension langsam zu einer wässrigen Natriumhydrogensulfit/Hydrogencarbonatlösung gegeben. Die organische Phase wurde abgetrennt und die wäßrige Phase mit Dichlormethan extrahiert. Die vereinigten organischen Phasen wurden getrocknet und anschließend das Lösungsmittel unter vermindertem Druck entfernt.
Ausbeute: 7,8 g leicht gelblicher Feststoff (90 % d. Th.)

### Beispiel 10

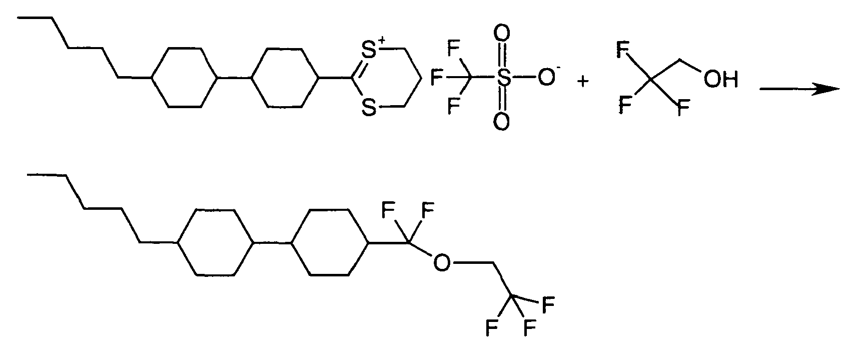

3,367 g des in Beispiel 2 erhaltenen Dithianyliumtrifluormethansulfonat wurden in 30 ml Dichlormethan bei -70°C vorgelegt und mit einer Mischung aus 1,128 ml Triethylamin und 0,737 g Trifluorethanol in 3 ml Dichlormethan versetzt, wobei ein farbloser Feststoff ausfiel. Man ließ 1 h bei -70°C rühren, versetzte mit HF/Pyridin (50 proz., entsprechend 66,97 mmol) ließ weitere 5 min rühren und ließ dann 1,715 ml Brom in 15 ml Tetrachlorkohlenstoff zutropfen. Es wurden weitere 10 ml Dichlormethan zugegeben. Man ließ noch 60 min rühren, ließ den Ansatz auf Raumtemperatur erwärmen und gab die gelbe Lösung unter Rühren vorsichtig auf 75 ml gesättigte Natriumhydrogencarbonatlösung. Nach dem Abklingen der Gasentwicklung wurde bis zur schwach alkalischen Reaktion Natriumhydrogencarbonat hinzugegeben, die organische Phase abgetrennt und die wässrige Phase dreimal mit je 30 ml Dichlormethan extrahiert. Die vereinigten organischen Phasen wurden mit Wasser gewaschen, über Natriumsulfat getrocknet und das Lösungsmittel im Vakuum entfernt. Der Rückstand wurde mit n-Hexan über Kieselgel filtriert. Das Rohprodukt wurde zweimal aus Pentan bei -78°C umkristallisiert.

Das Produkt wurde in Gegenwart von basischem Aluminiumoxid und Kupferpulver in Pentan über Nacht gerührt, mit Pentan über Kieselgel filtriert und aus Pentan bei -78°C auskristallisiert.

| | | |
|---|---|---|
| Ausbeute: | 445,0 mg (kristallisiertes Produkt) | |
| | ¹⁹F-NMR (235 MHz, CDCl₃) | |
| | δ = -80,50 ppm (d, ³J = 7,0 Hz, 2 F, CF₂O), - 72,47 (m, 3F, CF₃) | |
| | Schmelzpunkt: | 19°C |
| | Klärpunkt: | 72°C |

### Beispiel 11

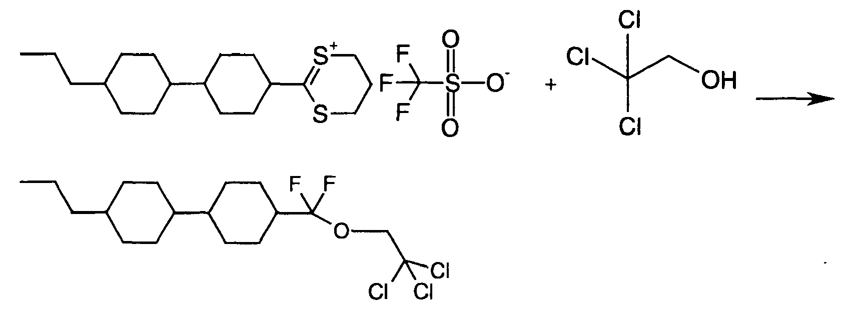

10,0 g des Dithianyliumtrifluormethansulfonats der oben gezeigten Formel, das analog dem in Beispiel 2 eingesetzten Dithianyliumtriflat hergestellt worden war, wurden bei -70°C vorgelegt, 150 ml Dichlormethan hinzugegeben und eine Mischung aus 8,761 ml Triethylamin und 6,053 ml Trichlorethanol in 10 ml Dichlormethan zugetropft, wobei sich die Lösung sofort gelb färbte und ein farbloser Feststoff ausfiel. Nach beendeter Zugabe ließ man 1 h bei -70°C rühren und versetzte dann mit HF/Pyridin (50 proz., entsprechend 210,670 mmol). Nach 5 min wurden 30,118 g 1,3-Dibromdimethylhydantoin, suspendiert in 50 ml Dichlormethan, portionsweise innerhalb von ca. 20 min hinzugegeben. Man ließ noch 2 h rühren, ließt den Ansatz auf -30°C erwärmen und gab die orangefarbene Suspension unter Rühren auf eine mit Natriumhydrogencarbonat gesättigte Mischung aus 300 ml gesättigter Natriumhydrogencarbonatslösung und 50 ml Natriumhydrogensulfitlsöung. Die organische Phase wurde abgetrennt und die wässrige Phase zweimal mit je 100 ml Dichlormethan extrahiert. Die vereinigten organischen Phasen wurden mit gesättigter Natriumchloridlösung gewaschen, über Natriumsulfat getrocknet und das Lösungsmittel im Vakuum entfernt. Der Rückstand wurde mit Hexan über Kieselgel filtriert. Das Rohprodukt wurde zur weiteren Reinigung in Ether gelöst, über Nacht in Gegenwart von Kupferpulver gerührt und erneut mit Hexan über Kieselgel filtriert. man erhielt ein schwach gelbes Öl, das langsam durchkristallisierte. Das Rohprodukt wurde zweimal aus Pentan bei - 20°C umkristallisiert.

| | | |
|---|---|---|
| Ausbeute: | 2,498 g (29,2 %) | |
| | ¹⁹F-NMR (235 MHz, CDCl₃) | |
| | δ = -81,18 ppm (d, ³J = 8,2 Hz, 2 F, CF₂O) | |
| | Schmelzpunkt: | 47°C |

### Beispiel 12

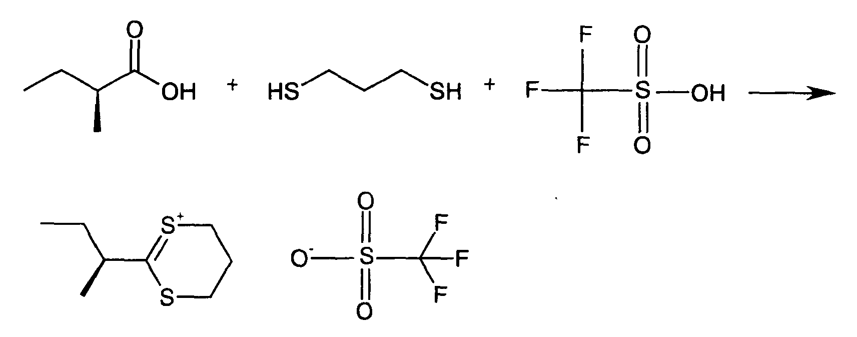

5 g (+)-S-2-Methylbuttersäure und 5,352 ml Dimercaptopropan wurden vorgelegt und 12,632 ml Trifluormethansulfonsäure zugetropft. Anschließend wurde 30 min auf 120°C erhitzt. Man ließ erkalten, gab 70 ml Ether hinzu und kühlte unter Rühren auf -78°C. Die Mutterlauge wurde bei derselben Temperatur mit einer Tauchfritte abgesaugt. Der schwach gelbe Feststoff wurde auf Raumtemperatur erwärmt, wobei dieser schmolz. Das so erhaltene gelbe Öl wurde mit 20 ml Ether versetzt und erneut unter kräftigem Rühren ausgefroren und abgesaugt. Man erhielt einen schwach gelben Feststoff, der über Nacht auf Trockeneis gelagert und ohne weitere Aufreinigung umgesetzt wurde.

### Beispiel 13

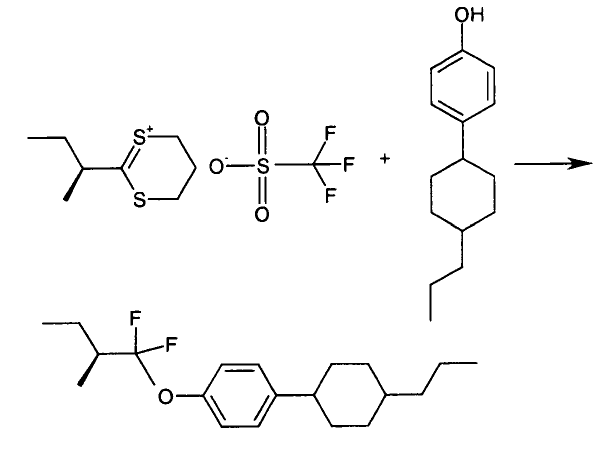

7,78 g des in Beispiel 12 hergestellten Triflats wurden in 50 ml Dichlormethan bei -70°C vorgelegt und langsam eine Lösung von 10,48 g des Phenols und Triethylamin (1,2 Äquivalente bezogen auf das Phenol) in 75 ml Dichlormethan zugetropft. Man ließ noch 1 h rühren, gab 19,96 ml Triethylamin-trishydrofluorid hinzu und ließ anschließend eine Lösung von 6,147 ml Brom in 30 ml Dichlormethan innerhalb von 1 h zutropfen. Man ließ noch 90 min rühren, ließ den Ansatz auf -20°C erwärmen und gab die Lösung auf 500 ml 1 M eiskalte Natronlauge. Die wässrige Phase wurde abgetrennt und dreimal mit Dichlormethan extrahiert. Die vereinigten organischen Phasen wurden in Gegenwart von Celite 30 min kräftig gerührt, filtriert, zweimal mit Wasser gewaschen und über Natriumsulfat getrocknet. Das Lösungsmittel wurde im Vakuum entfernt und der Rückstand mit n-Hexan über Kieselgel filtriert. Man erhielt ein farbloses Öl, das zweimal aus n-Pentan bei -78°C auskristallisiert wurde.

| | | |
|---|---|---|
| Ausbeute: | 3,390 g (43,5 %) des zweimal kristallisierten Produkts | |
| | ¹⁹F-NMR (235 MHz, CDCl₃) δ = 78,26 ppm (dd, ³J = 9,1 Hz, ³J = 11,5 Hz, 2 F, CF₂O) | |
| | Schmelzpunkt: | 33°C |

## Patentansprüche

1. Verfahren zur Herstellung von Verbindungen mit mindestens einer -CF₂-O-Brücke im Molekül, wobei zunächst ein Bis(alkylthio)carbeniumsalz in Gegenwart einer Base mit einer mindestens eine Hydroxylgruppe aufweisenden Verbindung umgesetzt wird und anschließend, vorzugsweise in situ, mit einem Fluorierungsmittel und einem Oxidationsmittel zur Verbindung mit mindestens einer -CF₂-O-Brücke im Molekül oxidativ fluoriert wird.

2. Verfahren nach Anspruch 1, wobei das Bis(alkylthio)carbeniumsalz eine Struktur der Formel I 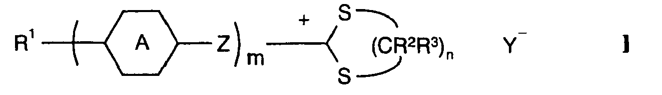 aufweist, wobei bedeutet: 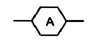 jeweils unabhängig voneinander cis- oder trans-1,4-Cyclohexylen, 1,4-Phenylen oder 1,4-Cyclohex-3-enylen, wobei diese Gruppen auch ein- oder zweifach substituiert sein können durch Halogen, insbesondere Fluor, Pseudohalogen, -OCF₃, -OCHF₂,
R¹ einen unsubstituierten, einen einfach durch -CN oder -CF₃ oder einen mindestens einfach durch Halogen substituierten linearen oder verzweigten Alkyl- oder Alkoxyrest mit 1 bis 12 C-Atomen, einen linearen oder verzweigten Oxyalkyl-, Alkenyl- oder Alkenyloxyrest mit 2 bis 12 C-Atomen oder einen linearen oder verzweigten Oxalkenylrest mit 3 bis 12 C-Atomen, wobei in diesen Resten auch eine oder mehrere CH₂-Gruppen jeweils unabhängig voneinander durch -O-, -S-, -CO-, -CO-O-, -O-CO- oder-O-CO-O- so ersetzt sein können, daß Heteroatome nicht direkt miteinander verknüpft sind, Halogen,
Z jeweils unabhängig voneinander eine Einfachbindung, eine -CH₂-CH₂-, -CF₂-CF₂-, -CF₂-CH₂-, -CH₂-CF₂-, -CF=CF-, -CH=CH-, -C≡C-, -CO-O-, -O-CO-, -CF₂-O-, -O-CF₂-Gruppe,
-S-(CR²R³)ₙ-S- eine Brücke aus zwei Schwefelatomen, die durch eine Kohlenstoffkette mit zwei oder drei Kohlenstoffatomen verbunden ist, wobei die Kohlenwasserstoffbrücke auch einen oder mehrere Substituenten R², R³ tragen kann, insbesondere Alkyl- oder Alkenylgruppen, wobei die Reste R², R³ auch gemeinsam eine Cycloalkylgruppe oder Arylgruppe ausbilden können,
m einen ganzzahligen Wert zwischen 0 und 6
n 2 oder 3
Y ein beliebiges Anion.

3. Verfahren nach Anspruch 1, wobei das Bis(alkylthio)carbenium-Salz ein nicht- oder schwach-koordinierendes Anion Y⁻ aufweist, das insbesondere ausgewählt ist aus der Gruppe, die gebildet ist von Tetrafluoroborat, Hexafluorophosphat, Perchlorat sowie Perfluoralkylsulfonat, insbesondere Trifluormethansulfonat.

4. Verfahren nach einem der Ansprüche 1 bis 3, wobei die mindestens eine Hydroxylgruppe enthaltende Verbindung ein Phenol ist, das gegebenenfalls substituiert sein kann, insbesondere in 4-Stellung eine polare Gruppe, bevorzugt -F, -Cl, -CN, -NCS, -OCF₃, -OCH₂F, oder einen Alkyl-, Cycloalkyl- oder Phenylrest trägt, der gegebenenfalls wiederum durch Alkyl-, Cycloalkyl- oder Phenylreste substituiert sein kann, wobei weiter ein bis drei Wasserstoffatome dieser Gruppen jeweils auch durch Fluor- oder Chloratome ersetzt sein können und zwischen diesen Resten auch eine -CH₂-CH₂-, -CF₂-CF₂-, -CF₂-CH₂-, -CH₂-CF₂-, -CF=CF-, -CH=CH-, -C≡C-, -C(O)O-, -O-CO-, -CF₂-O-, -O-CF₂-, -CH₂-O- oder -O-CH₂-Gruppe angeordnet sein kann.

5. Verfahren nach einem der Ansprüche 1 bis 4, wobei das Oxidationsmittel eine Verbindung ist, die Haloniumäquivalente freisetzt, insbesondere ausgewählt ist aus der Gruppe, die gebildet ist von Dimethyldibromhydanthoin, N-Bromsuccinimid, N-Jodsuccinimid, Brom, SOCl₂, SO₂ClF, Nitrosonium- und Nitroniumsalze sowie Chloramin T.

6. Verfahren nach einem der Ansprüche 1 bis 5, wobei das Fluorierungsmittel ausgewählt ist aus der Gruppe, die gebildet ist von aliphatischen und aromatischen Amin-Fluorwasserstoff-Komplexen, insbesondere ausgewählt ist aus der Gruppe, die gebildet ist von Pyridin-Fluorwasserstoff-Komplexen, NEt₃•3HF, Melamin•HF, Polyvinylpyridin•HF.

7. Bis-(alkylthio)carbeniumsalz der Formel I 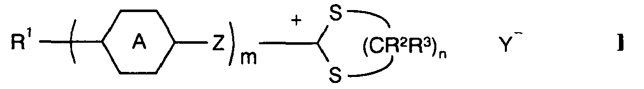 wobei R¹, 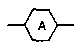 und -S-(CR²R³)-S-, Y⁻ sowie m und n die in Anspruch 2 angegebene Bedeutung aufweisen.

8. Dithioorthoester der Formel II 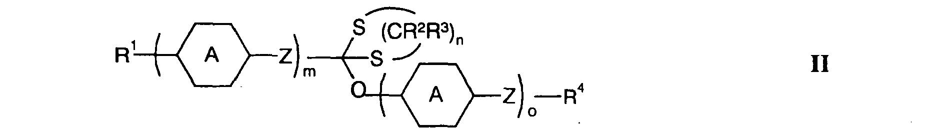 wobei R¹, R², R³, Z, 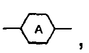 m, n die gleiche Bedeutung haben wie in Anspruch 2 angegeben und R⁴ die gleiche Bedeutung hat wie für R¹ in Anspruch 2 angegeben sowie zusätzlich -H, -F, -Cl, -CN, -NCS, -OCF₃ oder -OCHF₂ bedeuten kann, und o ein ganzzahliger Wert zwischen 0 und 6 ist.

9. Dithioorthoester nach Anspruch 8, wobei benachbart zum Sauerstoff der Dithioorthoesterfunktion eine 1,4-Phenylengruppe angeordnet ist.

## Claims

1. Process for the preparation of compounds having at least one -CF₂O- bridge in the molecule, which comprises initially reacting a bis(alkylthio)-carbenium salt with a compound containing at least one hydroxyl group in the presence of a base followed by oxidative fluorination, preferably in situ, with a fluorinating agent and an oxidizing agent to form the compound having at least one -CF₂O- bridge in the molecule.

2. Process according to Claim 1, wherein the bis(alkylthio)carbenium salt has a structure of the formula (I) 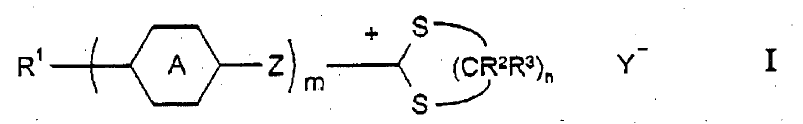 in which: 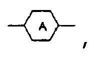 in each case independently of one another, is cis- or trans-1,4-cyclohexylene, 1,4-phenylene or 1,4-cyclohex-3-enylene, where these groups may also be monosubstituted or disubstituted by halogen, in particular fluorine, pseudohalogen, -OCF₃ or -OCHF₂,
R¹ is a linear or branched alkyl or alkoxy radical having 1 to 12 carbon atoms which is unsubstituted, monosubstituted by -CN or -CF₃ or at least monosubstituted by halogen, a linear or branched oxyalkyl, alkenyl or alkenyloxy radical having 2 to 12 carbon atoms or a linear or branched oxalkenyl radical having 3 to 12 carbon atoms, where, in addition, one or more CH₂ groups in these radicals may, in each case independently of one another, be replaced by -0-, -S-, -CO-, -CO-O-, -O-CO- or -O-CO-O- in such a way that heteroatoms are not linked directly to one another, or halogen,
Z is, in each case independently of one another, a single bond, a -CH₂-CH₂-, -CF₂-CF₂-, -CF₂-CH₂-, -CH₂-CF₂-, -CF=CF-, -CH=CH-, -C≡C-, -CO-O-, -O-CO-, -CF₂-O- or -O-CF₂- group,
-S-(CR²R³)ₙ-S- is a bridge consisting of two sulfur atoms which are linked by a carbon chain having two or three carbon atoms, where the hydrocarbon bridge may also carry one or more substituents R² and R³, in particular alkyl or alkylene groups, where, in addition, the radicals R² and R³ may together form a cycloalkyl group or aryl group,
m is an integer from 0 to 6,
n is 2 or 3, and
Y⁻ is any desired anion.

3. Process according to Claim 1, wherein the bis(alkylthio)carbenium salt contains a noncoordinating or weakly coordinating anion Y⁻, in particular selected from the group consisting of tetrafluoroborate, hexafluorophosphate, perchlorate and perfluoroalkylsulfonate, in particular trifluoromethanesulfonate.

4. Process according to one of Claims 1 to 3, wherein the compound containing at least one hydroxyl group is a phenol which may optionally be substituted, and in particular carries a polar group, preferably -F, -Cl, -CN, -NCS, -OCF₃ or -OCH₂F, or an alkyl, cycloalkyl or phenyl radical in 4-position which in turn may be substituted by alkyl, cycloalkyl or phenyl radicals, where, in addition, one to three hydrogen atoms of these groups may in each case also be replaced by fluorine or chlorine atoms and a -CH₂-CH₂-, -CF₂-CF₂-, -CF₂-CH₂-, -CH₂-CF₂-, -CF=CF-, -CH=CH-, -C≡C-, -C(O)O-, -O-CO-, -CF₂-O-, -O-CF₂-, -CH₂-O- or -O-CH₂- group may also be present between these radicals.

5. Process according to one of Claims 1 to 4, wherein the oxidizing agent is a compound which releases haloniuin equivalents, and is in particular selected from the group consisting of dimethyldibromohydanthoin, N-bromosuccinimide, N-iodosuccinimide, bromine, SOCl₂, SO₂ClF, nitrosonium and nitronium salts and Chloramin T.

6. Process according to one of Claims 1 to 5, wherein the fluorinating agent is selected from the group consisting of aliphatic and aromatic amine/hydrogen fluoride complexes, and is in particular selected from the group consisting of pyridine/hydrogen fluoride complexes, NEt₃·3HF, melamine·HF and polyvinylpyridine·HF.

7. Bis(alkylthio)carbenium salt of the formula I 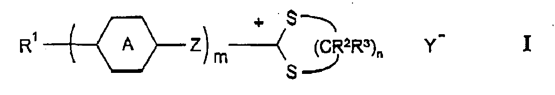 in which R¹, 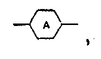 -S- (CR²R³) -S-, Y⁻, m and n are as defined in Claim 2.

8. Dithioorthoesters of the formula (II) 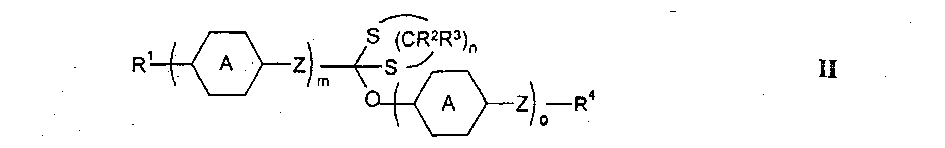 in which R¹, R², R³, Z, 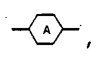 m and n are as defined in Claim 2, and R⁴ is as defined for R¹ in Claim 2, and, in addition, may be -H, -F, -Cl, -CN, -NCS, -OCF₃ or -OCHF₂, and o is an integer between 0 and 6.

9. Dithioorthoester according to Claim 8, wherein a 1,4-phenylene group is present adjacent to the oxygen atom of the dithioorthoester functionality.

## Revendications

1. Procédé de fabrication de composés ayant au moins un pont -CF₂-O- dans la molécule, où l'on fait réagir d'abord un sel de bis(alkylthio)carbénium en présence d'une base avec au moins un composé présentant un groupe hydroxyle, qui est ensuite fluoré par oxydation, de préférence in situ, avec un agent fluorant et un agent d'oxydation en un composé ayant au moins un pont -CF₂-O- dans la molécule.

2. Procédé selon la revendication 1, dans lequel le sel de bis(alkylthio)carbénium présente une structure de formule I 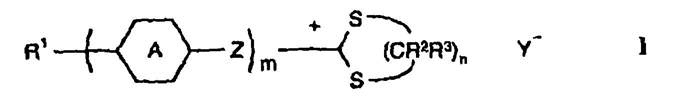 dans laquelle: 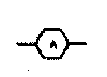 représente, indépendamment l'un de l'autre, le cis- ou trans-1,4-cyclohexylène, le 1,4-phénylène ou le 1,4-cyclohex-3-énylène, où ces groupes peuvent être mono- ou disubstitués par un halogène, en particulier le fluor, un pseudohalogène, -OCF₃, -OCHF₂,
R¹ représente un radical alkyle ou alcoxy linéaire ou ramifié non substitué, monosubstitué par -CN ou -CF₃ ou au moins monosubstitué par un halogène, ayant de 1 à 12 atomes de C, un radical oxyalkyle, alcényle ou alcényloxy linéaire ou ramifié, ayant de 2 à 12 atomes de C ou un radical oxalcényle linéaire ou ramifié ayant de 3 à 12 atomes de C, où, dans ces radicaux, également un ou plusieurs groupes CH₂ peuvent être remplacés indépendamment les uns des autres par -O-, -S-, -CO-, -CO-O-, -O-CO- ou -O-CO-O- de manière que les hétéroatomes ne soient pas directement reliés les uns aux autres, un halogène,
Z représente, indépendamment l'un de l'autre, une simple liaison, un groupe -CH₂-CH₂-, -CF₂-CF₂-, -CF₂-CH₂-, -CH₂-CF₂-, -CF=CF-, -CH=CH-, -C≡C-, -CO-O-, -O-CO-, -CF₂-O-, -O-CF₂-,
-S-(CR²R³)ₙ-S- représente un pont constitué de deux atomes de soufre, qui est relié avec deux ou trois atomes de carbone par une chaîne carbonée, où le pont hydrocarbure peut aussi porter un ou plusieurs substituants R², R³, en particulier des groupes alkyles ou alcényles, où les radicaux R², R³ peuvent aussi former conjointement un groupe cycloalkyle ou un groupe aryle,
m représente un nombre entier entre 0 et 6,
n représente 2 ou 3,
Y⁻ représente un anion quelconque.

3. Procédé selon la revendication 1, dans lequel le sel de bis (alkylthio) carbénium présente un anion Y⁻ non coordonné ou faiblement coordonné, qui est notamment choisi dans le groupe qui est formé par le tétrafluoroborate, l'hexafluoro-phosphate, le perchlorate et le perfluoroalkylsulfonate, en particulier le trifluorométhanesulfonate.

4. Procédé selon l'une des revendications 1 à 3, dans lequel le composé contenant au moins un groupe hydroxyle est un phénol, qui peut être substitué le cas échéant, porte notamment en position 4 un groupe polaire, de préférence -F, -Cl, -CN, -NCS, -OCF₃, - OCH₂F, ou un radical alkyle, cycloalkyle ou phényle, qui peut être le cas échéant de nouveau substitué par des radicaux alkyles, cycloalkyles ou phényles, où également un à trois atomes d'hydrogène de ces groupes peuvent être respectivement remplacés également par des atomes de fluor ou de chlore et où un groupe -CH₂-CH₂-, -CF₂-CF₂-, -CF₂-CH₂-, -CH₂-CF₂-, -CF=CF-, -CH=CH-, -C≡C-, -C(O)O-, -O-CO-, -CF₂-O-, -O-CF₂-, -CH₂-O- ou -O-CH₂-.

5. Procédé selon l'une des revendications 1 à 4, où l'agent d'oxydation est un composé qui libère des équivalents halonium, choisis en particulier dans le groupe constitué par la diméthyldibromohydanthoïne, le N-bromosuccinimide, le N-iodosuccinimide, le brome, SOCl₂, SO₂ClF, les sels de nitrosonium et de nitronium et la chloramine T.

6. Procédé selon l'une des revendications 1 à 5, où l'agent de fluoration est choisi dans le groupe constitué par les complexes amine - fluorure d'hydrogène aliphatiques et aromatiques, choisis en particulier dans le groupe constitué par les complexes de pyridine - fluorure d'hydrogène, NEt₃·3HF, mélamine **•** HF, polyvinylpyridine • HF.

7. Sel de bis(alkylthio)carbénium de formule I 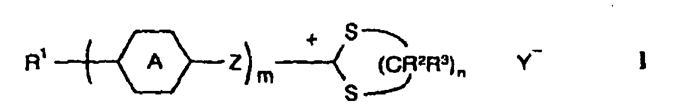 dans laquelle R¹, 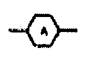 et -S- (CR²R³) -S-, Y⁻ et m et n ont la signification indiquée dans la revendication 2.

8. Dithioorthoester de formule II 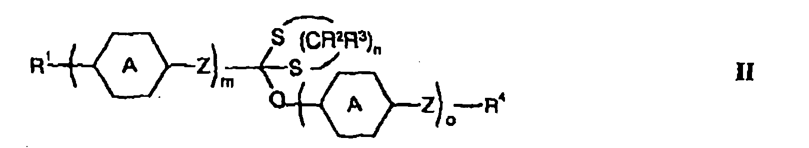 dans laquelle R¹, R², R³, Z, 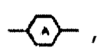 m et n ont la même signification que celle indiquée dans la revendication 2 et R⁴ a la même signification que celle indiquée pour R¹ dans la revendication 2 et peut signifier, en plus, -H, -F, -Cl, -CN, -NCS, -OCF₃ ou - OCHF₂, et o est un nombre entier entre 0 et 6,

9. Dithioorthoester selon la revendication 8, où un groupe 1,4-phénylène est disposé de manière adjacente à l'oxygène de la fonction dithioorthoester.
